# EUROPEAN PATENT SPECIFICATION

(11) **EP 4 110 207 B1**
(45) Date of publication and mention of the grant of the patent: **21.10.2026**
(21) Application number: 21711994.0
(22) Date of filing: 24.02.2021
(51) Int. Cl.: A61B 17/34

(54) **FLEXIBLE INTRAOSSEOUS OBTURATOR**
FLEXIBLER INTRAOSSALER OBTURATOR
OBTURATEUR INTRA-OSSEUX SOUPLE

(30) Priority: 28.02.2020 US 202062983434 P
(43) Date of publication of application: 04.01.2023
(73) Proprietor: Bard Access Systems, Inc., Salt Lake City, UT 84116 (US)
(72) Inventor: LINDEKUGEL, Eric, W., Salt Lake City, UT 84106 (US)
(74) Representative: Hoffmann Eitle
(86) International application number: PCT/US2021/019388
(87) International publication number: WO 2021/173649

(56) References cited:
- WO-A1-2019/164990
- WO-A2-2004/073500
- US-A1- 2018 256 209

## Description

This application claims the benefit of priority to U S. Provisional Application No. 62/983,434, filed February 28, 2020.

### SUMMARY OF THE INVENTION

Claims 1 and 7 define the invention and dependent claims disclose embodiments. No surgical methods are claimed per se. Current intraosseous access devices include rigid, stainless steel obturators to prevent bone fragments and other tissues from blocking the needle lumen during a placement event. Obturators in intraosseous ("1.0. ") needles prevent a hollow needle from coring the bone as it is drilled through the bone. The obturator is disposed within the needle lumen and extends flush with the needle bevel. This blocks the inner lumen of the needle and prevents the needle from becoming blocked by bone fragments as it is drilled through the bone.

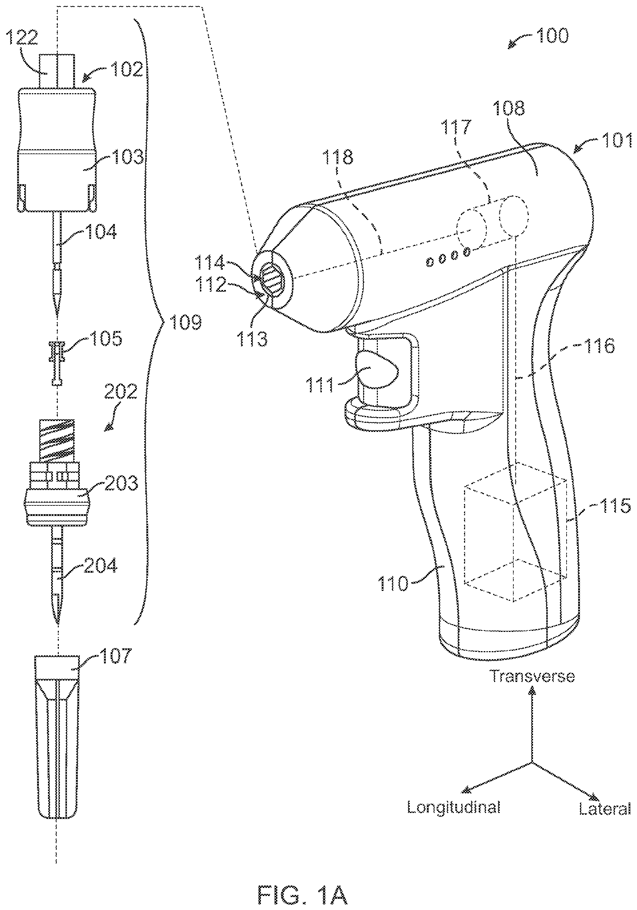

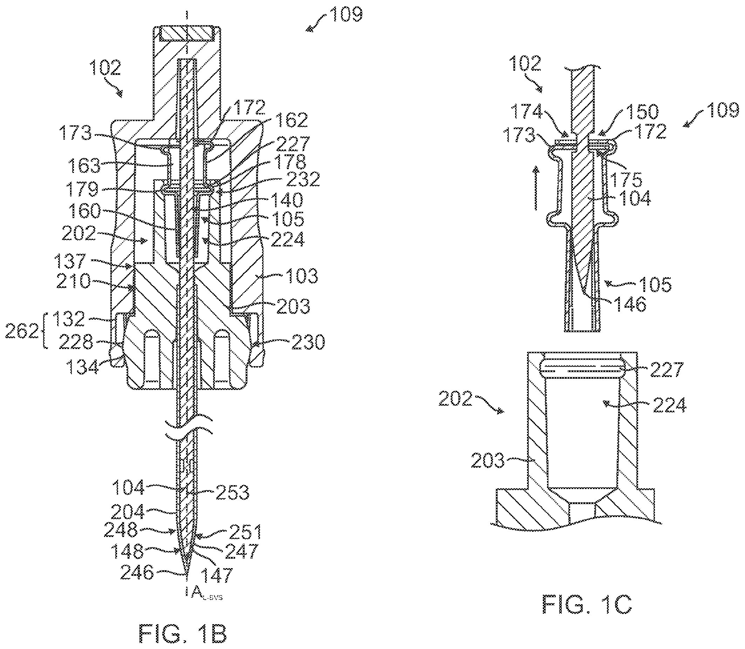

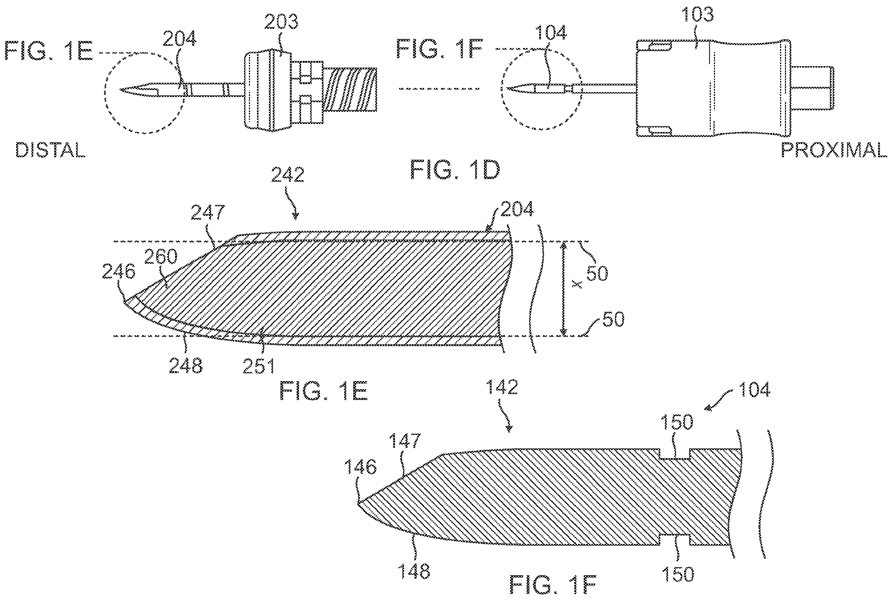

While stainless steel obturators are not intentionally provided with a distal cutting edge, the tip of the obturator is ground flush with the bevel of the needle to prevent any pockets forming that might collect bone fragments and the like. As shown for example, in FIGS. 1A-1C, this results in a sharpened tip 146 that requires a tip safety mechanism 105 to prevent accidental needle stick injuries once removed from the needle 204. Further the stainless steel obturator requires a concentric groove, or recess 150, to allow the tip safety mechanism to lock on to the tip of the obturator 104. This requires added complexity and cost in manufacturing intraosseous access devices that include safety shields and structures to engage the shields.

Briefly summarized, embodiments disclosed herein are directed to apparatus and methods for a flexible obturator for use with an intraosseous device, which resolves the previously stated problems. Disclosed herein is an access assembly for use with an intraosseous access device including, a needle including a rounded distal region communicating with a beveled distal opening, and an obturator formed of a flexible material, the obturator including a radially symmetrical elongate body designed for insertion in a lumen of the needle. The elongate body is deformable when disposed in the lumen of the needle to conform to fit the shape of an inner profile of the distal lumen. The elongate body further includes a needle hub configured to support the needle and define a hub lumen that communicates with the needle lumen, and an obturator hub configured to support the obturator, wherein a distal tip of the obturator extends through the needle lumen and extends distally of a distal tip of the needle when the obturator hub engages the needle hub.

In some embodiments, the obturator comprises a distal surface extending perpendicular to a longitudinal axis. The obturator comprises a curved distal surface. In some embodiments the elongate body further includes a beveled distal surface configured to align with the beveled distal opening of the needle. The flexible material displays relatively low columnar strength, low shear strength and high compressive strength. The flexible material includes one of a plastic, polymer, thermoplastic, Polytetrafluoroethylene ("PTFE"), Polyethylene ("PE"), Polyurethane ("PET"), rubber, silicone, metal, alloy, or Nitinol.

In some embodiments the elongate body further includes a lumen clearing mechanism, configured to advance the obturator relative to the needle lumen to clear material from a distal portion of the needle lumen. The lumen clearing mechanism is automatically triggered when the obturator is removed from the needle lumen.

Also disclosed is a method of manufacturing an access assembly including, providing a needle supported by a needle hub and defining a lumen, providing an obturator supported by an obturator hub, the obturator comprising a radially symmetrical elongate body designed for insertion in a lumen of the needle, wherein the elongate body is deformable when disposed in the lumen of the needle to conform to fit the shape of an inner profile of the distal lumen, advancing the obturator through the needle lumen until a distal tip extends distally of a distal tip of the needle when the obturator hub engages the needle hub, trimming a distal portion of the obturator to provide a distal surface that is flush with a beveled distal surface of the needle.

In some embodiments the method further includes adhering the obturator to the obturator hub prior to advancing the obturator through the needle lumen. The obturator is formed of a flexible material that displays relatively low columnar strength, low shear strength and high compressive strength. The flexible material includes one of a plastic, polymer, thermoplastic, Polytetrafluoroethylene ("PTFE"), Polyethylene ("PE"), Polyurethane ("PU"), rubber, silicone, metal, alloy, or Nitinol. The obturator defines a radially symmetrical elongate body. Providing an obturator includes the obturator defining a distal surface extending perpendicular to a longitudinal axis of the obturator. Providing an obturator includes the obturator defining a radially symmetrical curved distal surface. In some embodiments the method further includes a lumen clearing mechanism, configured to advance the obturator relative to the needle lumen to clear material from a distal portion of the needle lumen. The lumen clearing mechanism is automatically triggered when the obturator hub is detached from the needle hub. WO2004/073500 A2, WO2019/164990 A1 and US2018/256209 A1 disclose devices of the prior art.

### DRAWINGS

A more particular description of the present disclosure will be rendered by reference to specific embodiments thereof that are illustrated in the appended drawings. It is appreciated that these drawings depict only typical embodiments of the disclosure and are therefore not to be considered limiting of its scope. Example embodiments will be described and explained with additional specificity and detail through the use of the accompanying drawings in which:
FIG. 1A illustrates an exploded view of an exemplary intraosseous access system, wherein an access assembly subset of the system is depicted slightly enlarged and in elevation, and a driver component is depicted in perspective, in accordance with embodiments disclosed herein;
FIG. IB illustrates a cross-sectional view of the access assembly of FIG. 1A, in accordance with embodiments disclosed herein;
FIG. 1C illustrates a cross-sectional view of the obturator tip and safety shield removed from the access assembly of FIG. 1A, in accordance with embodiments disclosed herein;
FIGS. 1D-1F illustrate close up detailed views of the access assembly of FIG. 1A, in accordance with embodiments disclosed herein;
FIG. 2 A illustrates an obturator assembly, in accordance with embodiments disclosed herein;
FIGS. 2B-2E illustrate side views and proximal end views of an obturator, in accordance with embodiments disclosed herein;
FIGS. 3A-3C illustrates an obturator assembly, in accordance with embodiments disclosed herein; and
FIGS. 3D-3E illustrate a cross-sectional view of an obturator and needle assembly, in accordance with embodiments disclosed herein;

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a needle disclosed herein includes a portion of the needle intended to be near a clinician when the needle is used on a patient. Likewise, a "proximal length" of, for example, the needle includes a length of the needle intended to be near the clinician when the needle is used on the patient. A "proximal end" of, for example, the needle includes an end of the needle intended to be near the clinician when the needle is used on the patient. The proximal portion, the proximal end portion, or the proximal length of the needle can include the proximal end of the needle; however, the proximal portion, the proximal end portion, or the proximal length of the needle need not include the proximal end of the needle. That is, unless context suggests otherwise, the proximal portion, the proximal end portion, or the proximal length of the needle is not a terminal portion or terminal length of the needle.

With respect to "distal," a "distal portion" or a "distal end portion" of, for example, a needle disclosed herein includes a portion of the needle intended to be near or in a patient when the needle is used on the patient. Likewise, a "distal length" of, for example, the needle includes a length of the needle intended to be near or in the patient when the needle is used on the patient. A "distal end" of, for example, the needle includes an end of the needle intended to be near or in the patient when the needle is used on the patient. The distal portion, the distal end portion, or the distal length of the needle can include the distal end of the needle; however, the distal portion, the distal end portion, or the distal length of the needle need not include the distal end of the needle. That is, unless context suggests otherwise, the distal portion, the distal end portion, or the distal length of the needle is not a terminal portion or terminal length of the needle.

As shown in FIG. 1 A, and to assist in the description of embodiments described herein, a longitudinal axis extends substantially parallel to an axial length of a needle 204 extending from the driver 101. A lateral axis extends normal to the longitudinal axis, and a transverse axis extends normal to both the longitudinal and lateral axes. Unless defined otherwise, all technical and scientific terms used herein have the same meaning as commonly understood by those of ordinary skill in the art.

The present disclosure generally relates to intraosseous ("IO") access devices, systems, and methods thereof. FIG. 1 A shows an exploded view of an exemplary intraosseous access system 100, with some components thereof shown in elevation and another shown in perspective. The intraosseous access system 100 can be used to penetrate skin and underlying hard bone for intraosseous access, such as, for example to access the marrow of the bone and/or a vasculature of the patient via a pathway through an interior of the bone.

In an embodiment, the system includes a driver 101 and an access assembly 109. The driver 101 can be used to rotate the access assembly 109 into a bone of a patient. In embodiments, the driver 101 can be automated or manual. In an embodiment, the driver 101 is an automated driver 108. For example, the automated driver 108 can be a drill that achieves high rotational speeds. The intraosseous access system 100 can further include an obturator assembly 102, a shield 105, and a needle assembly 202, which may be referred to, collectively, as the access assembly 109. In an embodiment, the obturator assembly 102 includes an obturator 104 and an obturator hub 103. In an embodiment, the obturator hub 103 is attached to the obturator 104 in any suitable manner (e.g., one or more adhesives or overmolding). The obturator hub 103 can be configured to interface with the driver 101.

In an embodiment, the shield 105 is configured to couple with the obturator 104.
The coupling can allow longitudinal movement between the obturator 104 and the shield 105, when the shield 105 is in a first operational mode. In a second operational mode, the longitudinal movement between the obturator 104 and the shield 105 can be prevented.

For example, in the first operational mode, the obturator 104 maintains the shield 105 in an unlocked state. The obturator 104 can then be moved to a position where the shield 105 is no longer maintained in the unlocked state, and the shield 105 may automatically transition to the second operational mode, i.e. a locked state, in which little or no longitudinal movement is permitted between the shield 105 and the obturator 104. In the second operational mode, the shield 105 can inhibit inadvertent contact with a distal tip of the obturator 104 and prevent accidental needle stick injuries. In an embodiment, the shield 105 may be configured to rotate relative to the obturator 104, about a longitudinal axis, one of the first operational mode or the second operational mode.

The automated driver 108 may take any suitable form. The driver 108 may include a handle 110 that may be gripped by a single hand of a user. The driver 108 may further include an actuator 111, e.g. trigger actuator, of any suitable variety via which a user may selectively actuate the driver 108 to effect rotation of a coupling interface 112. For example, the actuator 111 may comprise a button, as shown, or a switch or other mechanical or electrical element for actuating the driver 108. In an embodiment, the coupling interface 112 is formed as a socket 113 that defines a cavity 114. The coupling interface 112 can be configured to couple with the obturator hub 103. In an embodiment, the socket 113 includes sidewalls that substantially define a hexagonal cavity into which a hexagonal protrusion of the obturator hub 103 can be received. Other suitable connection interfaces are contemplated.

The automated driver 108 can include an energy source 115 of any suitable variety that is configured to energize the rotational movement of the coupling interface 112. For example, in some embodiments, the energy source 115 may comprise one or more batteries that provide electrical power for the automated driver 108. In other embodiments, the energy source 115 can comprise one or more springs (e.g., a coiled spring) or other biasing member that may store potential mechanical energy that may be released upon actuation of the actuator 111. The energy source 115 may be coupled with the coupling interface 112 in any suitable manner. For example, in an embodiment, the automated driver 108 includes an electrical, mechanical, or electromechanical coupling 116 to a gear assembly 117. In some embodiments, the coupling 116 may include an electrical motor that generates mechanical movement from electrical energy provided by an electrical energy source 115. In other embodiments, the coupling 116 may include a mechanical linkage that mechanically transfers rotational energy from a mechanical (e.g., spring-based) energy source 115 to the gear assembly 117. The automated driver 108 can include a mechanical coupling 118 of any suitable variety to couple the gear assembly 117 with the coupling interface 112. In other embodiments, the gear assembly 117 may be omitted.

In embodiments, the automated driver 108 can rotate the coupling interface 112, and thereby, can rotate the access assembly 109 at rotational speeds significantly greater than can be achieved by manual rotation of the access assembly 109. For example, in various embodiments, the automated driver 108 can rotate the access assembly 109 at speeds of between 200 and 3,000 rotations per minute (rpm). However, it will be appreciated that lesser or greater rotational speeds are also contemplated.

As shown in FIG. 1A, the needle assembly 202 includes a needle 204 and a needle hub 203 that is attached to the needle 204 in any suitable manner. The needle hub 203 can be configured to couple with the obturator hub 103 and may thereby be coupled with the driver 101. FIGS. IB -IF show further details of the access assembly 109. FIG. IB shows a cross-sectional view of the access assembly 109 with the needle hub 203 retained by the obturator hub 103. The obturator 104 is disposed within the needle and the shield 105 is in the unlocked position within the access assembly 109. FIG. 1C shows a cross-sectional view of the access assembly 109 with the obturator 104 removed from the needle and the shield in the second, locked operational mode. FIG. ID shows an exploded view of the access assembly 109. FIG. IE shows a close up, cross-sectional view of the needle 204. FIG. IF shows a close up, cross-sectional view of the obturator 104.

As discussed herein, the obturator 104 is formed of a rigid material, e g. stainless steel, to inhibit tissue and/or bone from entering a lumen of the needle 204 during an access event. Although not intentionally provided with a cutting distal edge, the rigid obturator 104 is ground flush with the beveled opening of the needle to provide a flush surface. Further the rigid material sufficiently rigid and strong to inhibit tissue and/or bone from entering a lumen of the needle 204 during an access event. As such, the obturator tip 146 is sufficiently sharpened to provide a risk of needle stick injuries, and the shield 105 is provided to engage the obturator 104, and prevent accidental needle stick injuries. Further details and embodiments of the intraosseous access system 100 can be found in WO 2018/075694, WO 2018/165334, WO 2018/165339, and US 2018/0116693.

FIG. IB depicts an early stage of an illustrative method of using the intraosseous access system 100, and is a cross-sectional view of the access assembly 109 in an assembled state with an exemplary tip protection device. It should be appreciated that other means of tip protection are also contemplated and within the scope of the invention. The access assembly 109 includes the obturator assembly 102, the shield 105, and the needle assembly 202. In some instances, the access assembly 109 will be preassembled, and thus may be removed from any suitable sterile packaging substantially in the configuration depicted in FIG. IB. In the illustrated assembled state, the keyed coupling interfaces 137, 210 of the obturator hub 103 and the needle hub 203, respectively, can cooperate to ensure that a predetermined relationship between the obturator 104 and the needle 204 is achieved. Thus, the keyed coupling interfaces 137, 210 can ensure that the obturator 104 defines a fixed angular orientation relative to the needle 204. The coupling interfaces 137, 210 may likewise maintain the fixed angular orientation during rotation of the access assembly 109 during an insertion event, e.g., during rotation of the access assembly 109 via the automated driver 108.

The distal face 147 of the obturator 104 is slightly recessed relative to the distal face 247 of the needle 204. Additionally, the distal faces 147, 247 of the obturator 104 and the needle 204, respectively, are substantially parallel to each other. In some embodiments, the obturator 104 does not cut either through skin or bone during an insertion event. In other embodiments, the distal faces 147, 247 may be substantially flush with each other. The obturator 104 can substantially fill or otherwise block passage into the lumen 251 of the needle 204. For example, in the illustrated embodiment, the distal face 147 of the obturator 104 is substantially the same size as an opening into a distal end of the lumen 251. In various embodiments, an area of the distal face 147 of the obturator 104 is no greater than 5, 10, 15, or 20 percent smaller than an area defined by an inner edge of the distal face 247 of the needle 204. The obturator 104 can inhibit or prevent tissue and/or bone material from entering and/or progressing into the lumen 251 of the needle 204.

The interior surface 253 of the needle 204 and an exterior surface of the obturator 104 can be complementarily shaped and/or otherwise configured to prevent or inhibit ingress of tissue, bone, and/or other matter. In further embodiments, a fit between the obturator 104 and the needle 204 can permit the obturator 104 to be readily removed from needle 204. For example, a snug fit, a loose fit, or a minimal gap may be provided between at least a portion between the obturator 104 and the needle 204. During assembly of the access assembly 109, the arms or projections 132 of the obturator hub 103 can be advanced over the skirt 228 of the needle hub 203. The snap interface or inward protrusions 134 of the projections 132 can grip an underside of the skirt 228 to maintain the obturator hub 103 and the needle hub 203 in a coupled state. The skirt 228 is shaped substantially as an outward protrusion, and the inner surface of the arm 132 substantially defines a recess into which the protrusion is received. In other embodiments, the protrusion/recess interface may be reversed. For example, the arm 132 may define a protrusion is received into a recess defined by the skirt 228 to couple the obturator hub 103 with the needle hub 203.

The projection 132 and the skirt 228 may collectively be referred to as a releasable engagement mechanism 262. The releasable engagement mechanism 262 may be configured to keep the obturator hub 103 and the needle hub 203 coupled together during general manipulation of the access assembly 109, such as during removal from packaging and/or coupling thereof with the automated driver 108. The releasable engagement mechanism 262 may, however, provide a relatively weak coupling that is capable of being released upon application of sufficient removal force to the obturator hub 103 in a proximal direction, relative to the needle hub 203. For example, the releasable engagement mechanism 262 may provide a coupling force that tends to keep the obturator hub 103 engaged with the needle hub 203. When a proximally directed force on the obturator hub 103 exceeds the coupling force of the releasable engagement mechanism 262, the releasable engagement mechanism 262 can disengage and permit the obturator hub 103 to be withdrawn from the needle hub 203. In various embodiments, the coupling force (i.e., the force that counteracts a proximally directed force on the obturator hub 103) can be no greater than about 0.25 (1.72), 0.5 (3.45), 0.75 (5.17), 1.0 (6.89), 1 .5 (10.34), or 2.0 (13.79) pounds (MPa).

In certain embodiments, the releasable engagement mechanism 262 provides a coupling force that is significantly lower than an embedding force between the needle 204 and a bone within which the needle 204 is inserted. The releasable engagement mechanism 262 can be configured to permit the obturator hub 103 to be decoupled from the needle hub 203, after the needle hub 203 has been introduced into the bone, by imparting a proximally directed force on the obturator hub 103 that is smaller in magnitude than a force imparted on the cannula 204 by the bone that maintains the cannula 204 positioned in the bone. Accordingly, in some embodiments, after introducing the access assembly 109 into the bone, a user may simply pull back, or proximally, on the obturator hub 103 with any amount of force that exceeds the coupling force of the releasable engagement mechanism 262, and the obturator hub 103 will automatically disengage from the needle hub 203. Further, the obturator hub 103 can be withdrawn from the needle hub 203 and the patient, and the needle 204 can remain in the bone. In some instances, the user can remove the obturator hub 103 from the needle hub 203 using a single hand after the access assembly 109 has been introduced into the bone. Other suitable arrangements of the releasable engagement mechanism 262 are contemplated.

When the access assembly 109 is in the assembled state, the shield 105 can be coupled with each of the obturator 104 and the needle hub 204 in an unlocked state, in which the arms 162, 163 are deflected outwardly away from the longitudinal axis. In particular, the proximal end 140 of the obturator 104, which can define a larger diameter than does the recess 150, can extend through an entirety of the shield 105. The proximal end 140 of the obturator 104 extends through the lateral extensions 172, 173 and the collar 160. This larger diameter region of the obturator 104 can maintain the shield 105 in the unlocked state to permit the obturator 104 to translate relative to the shield 105 in a proximal direction when the user desires to remove the obturator hub 103 from the needle hub 204.

When the shield 105 is in the unlocked state, the arms are deflected outwardly, which can seat or otherwise position the outward protrusions 178, 179 of the arms 162, 163 respectively within the groove 227 of the needle hub 203. The outward protrusions 178, 179 thus can cooperate with the groove 227 to maintain the shield 105 in a fixed longitudinal position relative to the needle hub 203 during the initial stages of withdrawal of the obturator 104 through the shield 105. In other embodiments, the groove 227 and the outward protrusions 178, 179 can be reversed. For example, in some embodiments, an inner surface of the needle hub 203 may define one or more inward protrusions, and the arms 162, 163 may define inward recesses into which the inward protrusions are received when the shield 105 is in the unlocked state (relative to the obturator 104) and in the coupled state relative to the needle hub 203.

FIG. 1C is another enlarged cross-sectional view of the access assembly 109 when the obturator 104 has been fully withdrawn from the needle hub 203. Prior to the depicted stage, the obturator 104 is withdrawn proximally by a sufficient amount to bring the recess 150 into the vicinity of the openings 174, 175. Due to the reduced diameter of the recess 150, the constricted portions of the openings 174, 175 fit into the recess 150 and the arms 162, 163 are thus permitted to automatically transition to their unbiased, non-deflected, or non-deformed state, i.e., the arms 162, 163 can resiliently return to a less bent or unbent state to automatically lock the shield 105 to the obturator 104.

When the shield 105 is in the locked state, portions of the lateral extensions 172, 173 that define the constricted portions of the openings 174, 175 enter into the recess 150 to secure the shield 105 to the obturator 104. When the shield 105 is locked to the obturator 104, movement of the shield 105 relative to the obturator 104 can be prevented or delimited in one or more directions (e.g., longitudinally and/or rotationally). In some embodiments, interference between the lateral extensions 172, 173 and proximal and distal faces of the recess 150, respectively, can delimit longitudinal movement of the shield 105 relative to the obturator 104. When the arms 162, 163 automatically transition to the locked state relative to the obturator 104, the arms 162, 163 substantially simultaneously decouple the shield from the needle hub 203. In particular, the inward movement of the arms 162, 163 causes the outward protrusions to exit the groove 227 of the needle hub 203. This frees the shield 105 to move relative to the needle hub 203, such as for proximal movement in the longitudinal direction to exit the lumen 224. The shield 105 naturally remains in the locked state relative to the obturator 104 and restricts access to the distal tip 146 of the obturator 104.

As shown in FIG. IE, which is an enlarged view of the obturator 104 within the needle 204, the distal portion of the needle lumen 251 defines an asymmetrical shape including a rounded portion 248 and a beveled face 247 including an opening 260 that communicates with the needle lumen 251. As such, the obturator 104 formed of rigid material, which is shown in isolation in FIG. IF, includes a distal portion 142 shaped to match the distal portion of the needle lumen 251. Further, the obturator 104 includes a longitudinal length to match the longitudinal length of the needle lumen 251 to align the shaped portion with the needle lumen 251. As such, different lengths of rigid obturators 104 must be produced to match different longitudinal lengths of needles 204 and requires a high degree of engineering accuracy to position the distal portions correctly when the access assembly 109 is assembled.

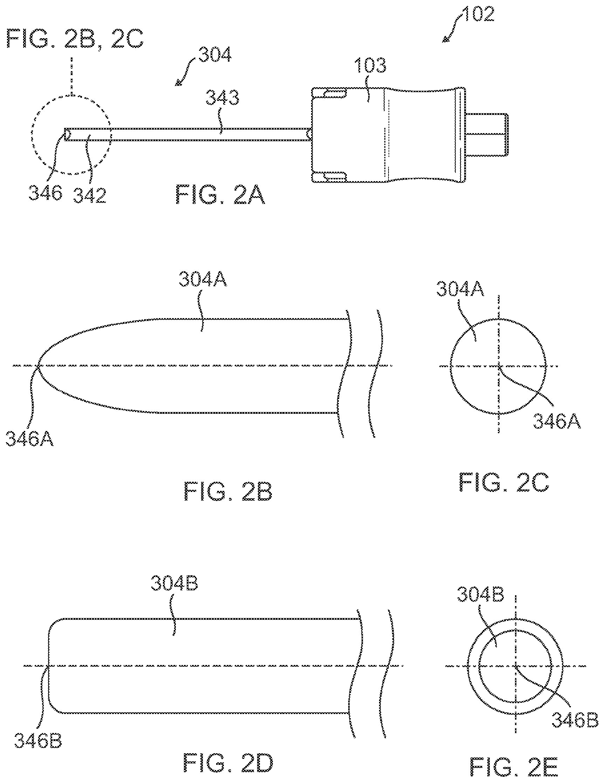

FIGS. 2A-2E show an exemplary embodiment of an obturator assembly 102 that includes an obturator 304 formed of a flexible material. The obturator 304 can define a substantially circular cross-section and includes a diameter sized to fit snugly within the inner diameter of the needle lumen 251. In an embodiment, a longitudinal length of the obturator 304 can be longer than the length of the obturator 104 that is used with needle 204. As such, when the obturator hub 103 engages the needle hub 203, for example as shown in FIG. IB, a distal tip 346 of the obturator 304 extends distally of the distal tip 246 of the needle 204.

In an embodiment, as shown in FIG. 2B, the distal tip 346A of the obturator 304A can define a rounded tip. In an embodiment, as shown in FIG. 2D, the distal tip 346B of the obturator 304B can define a squared off tip that includes a distal surface extending perpendicular to the longitudinal axis. Optionally, the edges of the distal surface 346B can be rounded or chamfered. As shown in FIGS. 2C and 2E, in an embodiment, the obturator 304A, 304B, defines a radially symmetrical cross-section extending from the longitudinal axis.

In an embodiment, an obturator 304 can be formed of a flexible plastic that displays elastic properties and can readily deform or bend when a force is applied and return to an undeformed shape when the force is removed. In an embodiment, the obturator 304 can be formed of a material that displays a combination of mechanical properties including a relatively high flexibility, high elasticity, as well as a high compressive strength. In an embodiment, the obturator 304 can be formed of a plastic, polymer, thermoplastic, Polytetrafluoroethylene ("PTFE"), Polyethylene ("PE"), Polyurethane ("PET"), rubber, silicone, metal, alloy, Nitinol or similar material.

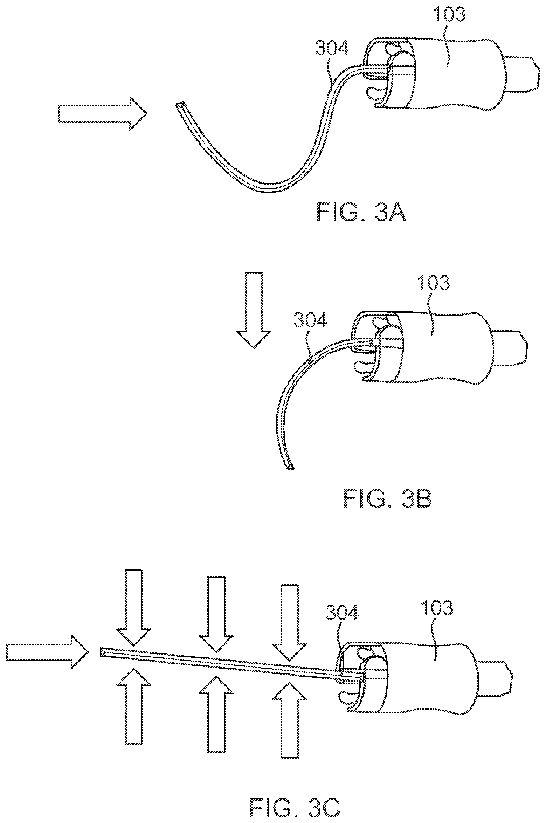

For example, as shown in FIG. 2A, the elongate obturator 304 extending from the obturator hub 103 is capable of self-supporting. However, as shown in FIG. 3 A, when a longitudinal force is applied, the obturator 304 can flex and deform thereby displaying relatively low columnar strength. When the force is removed, the obturator 304 displays elastic properties and returns to its original shape. Further, as shown in FIG. 3B, when a lateral force is applied, the obturator 304 can flex and deform thereby displaying relatively low sheer strength. When the force is removed, the obturator 304 displays elastic properties and returns to its original shape. However, as shown in FIG. 3C, the obturator 304 can display a relatively high compressive strength such that the obturator 304 resists equal and opposite forces applied to the material with relatively little or no deformation.

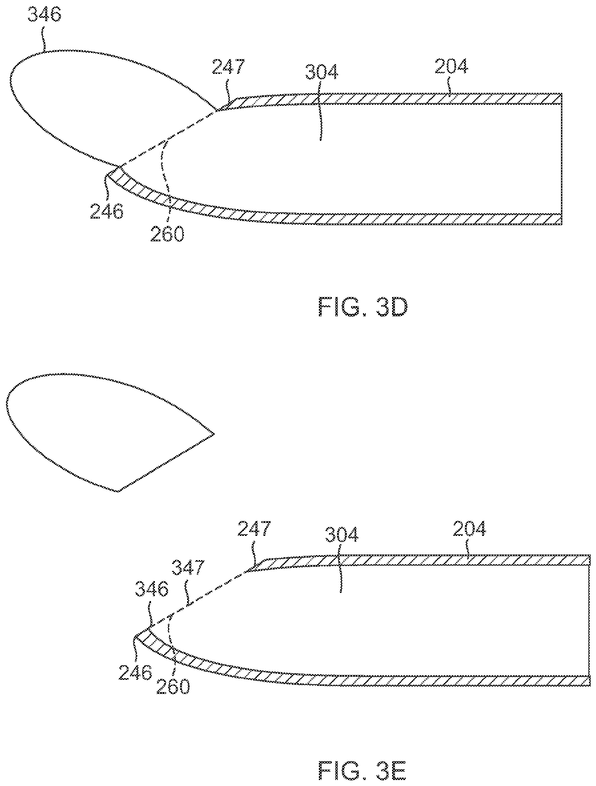

As shown in FIGS. 3D-3E, the flexible obturator 304 can advantageously conform to fit the shape of the distal portion of the needle lumen 251. Further, a distal portion 342 of the obturator 304 can flex to fit through the angled opening 260 such that a distal tip of the obturator 346 can extend distally of the distal tip 246 of the needle 204. As shown in FIG. 3E, the excess portion of the obturator 304 that extends through the opening 260, can be trimmed to provide a beveled surface 347 that lies flush with the beveled surface 247 of the needle 204. It will be appreciated that this provides a distal tip 346 that is then disposed proximally of the distal tip 246 of the needle.

It will be appreciated that the needle 204, formed of a rigid material, e.g. stainless steel, provides the necessary columnar strength and sheer strength to resist deformation of the needle 204/obturator 304 assembly. Further, the obturator 304, which is constrained within the needle lumen 251 displays sufficient compressive strength to resist a longitudinal force, e.g. to resist any bone fragments from being forced proximally into the needle lumen 251 during an insertion event. In an embodiment, the obturator 304 displays minimal compressive deformation that allows some retraction of the distal tip 346 into the needle lumen 251. In an embodiment, the elasticity of the obturator 304 then resumes the original, undeformed shape when the force is removed, i.e. when the needle penetrates the cortex layer of bone, and ejects any bone fragments or other material from the needle lumen 251.

Advantageously, due to the compressive strength of the obturator 304, the obturator 304 prevents bone material from entering the needle lumen during an insertion event. Further, when the obturator 304 is removed from needle 204, there is a reduced risk of needle stick injuries due to the flexible properties of the obturator 304. For example, when the flexible obturator 304, by itself, is subjected to longitudinal or lateral forces, the mechanical properties are configured allow the obturator 304 to flex, preventing breakage of the skin. Similarly, although the tip 346 of the obturator presents a sharpened point, the mechanical properties are configured allow the obturator 304 to deform, preventing breakage of the skin.

An exemplary method of manufacturing an access assembly 109, using the flexible obturator 304, is provided. An elongate cylinder of flexible material is provided to form a body 343 of the obturator 304 and defines a circular cross sectional shape. The outer diameter of the body 343 of the obturator 304 is configured to fit tightly within an inner diameter of the needle lumen 251. A proximal end of the obturator body 343 can be attached to an obturator hub 103 using welding, bonding, adhesive, or the like. A longitudinal length of the obturator 304 can be the same or longer than the longitudinal length of the needle lumen 251. As such, when the obturator hub 103 engages the needle hub 203, a distal tip of the obturator 304 can extend distally of the lumen opening 260. In an embodiment, the distal tip 346 can be cut or ground down to provide a beveled surface 347 to the obturator 304 that is flush with the beveled face 247, as shown in FIG. 3E.

Advantageously, the obturator 304 also provides a simplified manufacturing process. Initially, there is no requirement for a shield 105 since the flexibility of the obturator 304 prevents needle stick injuries. This simplifies the manufacture process, and provides a smaller access assembly 109. The manufacture of the obturator 304 is further simplified since there is no need for the formation of a recess 150 in the obturator 304 for engaging the shield 105, nor the annular groove 227 (FIG. IB) that retains the shield within the needle hub 203, or similar detailed structures. These detailed structures cannot be molded into place due to the size and precision required. Instead, these require additional steps to machine such features into place that add to the cost and complexity.

The manufacture of the obturator 304 is also simplified by only requiring a single base length of obturator that can easily be trimmed to suite any length of needle. For example, the flexible obturator 304 can negotiate the rounded region 248 and angled opening 260 of the needle 204, to be advanced distally of a distal tip 246 thereof. The flexible obturator 304 can then be trimmed to size and provide a beveled tip that is flush with the distal face 247 of the needle 204. This removes the need to form different obturators of different sizes to suit different needles and simplifies the manufacturing process. As shown in FIG. 2B, the axis lines 50 illustrate the inner diameter ( x ) of the needle lumen 251. The rounded region 248 and angled opening 260 can obstruct a rigid obturator from being advanced distally of a distal tip 246 of the needle 204. As such, rigid obturators can be pre-formed with a rounded portion 146 and beveled distal surface 147 to match the rounded portion 246 and beveled surface 247 of the needle lumen, prior to being assembled with the needle 204. As such different lengths of rigid obturators are required to suit different length needles.

Advantageously, the manufacturing process is further simplified in that a flexible obturator 304 can be attached to the obturator hub 103 before assembly with the needle 204 and before being trimmed. When fitting an obturator, e.g. obturator 104, to the needle 204 followed by attaching the obturator hub 103, the obturator can inadvertently adhere to additional structures, e.g. shield 105, needle hub 203, causing the access assembly to fail during use. Optionally, the flexible obturator 304 can also be attached to the obturator hub 103 after assembly with the needle 204. In an embodiment, the obturator tip 304 is trimmed prior to assembly with the needle 204. Advantageously, this prevents the tip of the needle being damaged or blunted during the trimming of the obturator after assembly with the needle.

In an embodiment, the access assembly 109 can further include a lumen clearing mechanism (not shown) that allows the obturator 304 to move slightly, along the longitudinal axis, relative to the needle 204. Advantageously, this allows a user to activate the lumen clearing mechanism after the needle 204 has been placed to advance the obturator 304 relative to the needle 204 and clear any material disposed within the needle lumen 251. As discussed herein, the flexible properties of the obturator 304 allow a distal portion to be advanced through the distal opening 260. In an embodiment, the lumen clearing mechanism is triggered automatically as part of the process of removing the obturator 304 from the needle 204. For example, the lumen clearing mechanism can be activated with the obturator hub 103 is detached from the needle hub 203.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein. The scope of the invention is defined by the claims.

## Claims

1. An access assembly (109) for use with an intraosseous access device, comprising:
a needle (204) including a rounded distal region (248) communicating with a beveled distal opening (260);
an obturator (104, 304) formed of a flexible material, the obturator (104, 304) comprising a radially symmetrical elongate body (343) designed for insertion in a lumen (251) of the needle;
a needle hub (203) configured to support the needle (204) and define a hub lumen that communicates with the needle lumen (251), and
an obturator hub (103) configured to support the obturator (104, 304), **characterised in that** a distal tip (146) of the obturator extends through the needle lumen (251) and extends distally of a distal tip (246) of the needle when the obturator hub (103) engages the needle hub (203); and
the elongate body (343) is deformable when disposed in the lumen (251) of the needle to conform to fit the shape of an inner profile of the distal lumen.

2. The access assembly according to claim 1, wherein the obturator (104, 304) comprises a distal surface (346B) extending perpendicular to a longitudinal axis.

3. The access assembly according to any claim of claims 1-2, wherein the obturator (104, 304) comprises a curved distal surface (346A).

4. The access assembly according to any claim of claims 1-3, wherein an excess portion of the obturator (304) that extends through the opening (260), is configured to be trimmed to provide a beveled distal surface (347) that lies flush with the beveled surface (247) of the needle (204).

5. The access assembly according to any claim of claims 1-4, wherein the flexible material displays relatively low columnar strength, low shear strength and high compressive strength; optionally wherein the flexible material includes one of a plastic, polymer, thermoplastic, Polytetrafluoroethylene ("PTFE"), Polyethylene ("PE"), Polyurethane ("PET"), rubber, silicone, metal, alloy, or Nitinol.

6. The access assembly (109) according to any claim of claims 1-6, further including a lumen clearing mechanism, configured to advance the obturator (104, 304) relative to the needle lumen (251) to clear material from a distal portion of the needle lumen (251); optionally wherein the lumen clearing mechanism is automatically triggered when the obturator (104, 304) is removed from the needle lumen (251).

7. A method of manufacturing an access assembly, comprising:
providing a needle (204) supported by a needle hub (203) and defining a lumen (251);
providing an obturator (104, 304) supported by an obturator hub (103), the obturator comprising a radially symmetrical elongate body (343) designed for insertion in a lumen (251) of the needle, **characterised in that** the elongate body (343) is deformable when disposed in the lumen of the needle (251) to conform to fit the shape of an inner profile of the distal lumen; and
the method further comprises:
advancing the obturator (104, 304) through the needle lumen (251) until a distal tip (146) extends distally of a distal tip (246) of the needle, when the obturator hub (103) engages the needle hub (203); and
trimming a distal portion (342) of the obturator to provide a distal surface (147) that is flush with a beveled distal surface (247) of the needle.

8. The method according to claim 7, further including adhering the obturator (104, 304) to the obturator hub (103) prior to advancing the obturator (104, 304) through the needle lumen (251).

9. The method according to either of claims 7 or 8, wherein the obturator (104, 304) is formed of a flexible material that displays relatively low columnar strength, low shear strength and high compressive strength; optionally wherein the flexible material includes one of a plastic, polymer, thermoplastic, Polytetrafluoroethylene ("PTFE"), Polyethylene ("PE"), Polyurethane ("PEI"), rubber, silicone, metal, alloy, or Nitinol.

10. The method according to any claim of claims 7-9, wherein providing an obturator (104, 304) includes the obturator (104, 304) defining a distal surface (346B) extending perpendicular to a longitudinal axis of the obturator.

11. The method according to any claim of claims 7-10, wherein providing an obturator (104, 304) includes the obturator (104, 304) defining a radially symmetrical curved distal surface (346A).

12. The method according to any claim of claims 7-11, further including a lumen clearing mechanism, configured to advance the obturator (104, 304) relative to the needle lumen (251) to clear material from a distal portion of the needle lumen (251).

13. The method according to claim 12, wherein the lumen clearing mechanism is automatically triggered when the obturator hub (103) is detached from the needle hub (203).

## Patentansprüche

1. Zugangsbaugruppe (109) zur Verwendung mit einer intraossären Zugangsvorrichtung, umfassend:
eine Nadel (204), einschließend einen abgerundeten distalen Bereich (248), der mit einer abgeschrägten distalen Öffnung (260) kommuniziert;
einen Obturator (104, 304), gebildet aus einem flexiblen Material, wobei der Obturator (104, 304) einen radial symmetrischen, länglichen Körper (343) umfasst, der für Einführung in ein Lumen (251) der Nadel ausgelegt ist;
eine Nadelnabe (203), die ausgebildet ist, um die Nadel (204) zu stützen, und ein Nabenlumen zu definieren, das mit dem Nadellumen (251) kommuniziert, und
eine Obturatornabe (103), die ausgebildet ist, um den Obturator (104, 304) zu stützen, **dadurch gekennzeichnet, dass** sich eine distale Spitze (146) des Obturators durch das Nadellumen (251) erstreckt und sich distal über eine distale Spitze (246) der Nadel hinaus erstreckt, wenn die Obturatornabe (103) in die Nadelnabe (203) eingreift; und
der längliche Körper (343) verformbar ist, wenn er im Lumen (251) der Nadel angeordnet ist, um sich an die Gestalt eines Innenprofils des distalen Lumens einzupassen.

2. Zugangsbaugruppe nach Anspruch 1, wobei der Obturator (104, 304) eine distale Oberfläche (346B) umfasst, die sich senkrecht zu einer longitudinalen Achse erstreckt.

3. Zugangsbaugruppe nach einem der Ansprüche 1-2, wobei der Obturator (104, 304) eine gekrümmte distale Oberfläche (346A) umfasst.

4. Zugangsbaugruppe nach einem beliebigen Anspruch der Ansprüche 1-3, wobei ein überstehender Abschnitt des Obturators (304), der sich durch die Öffnung (260) erstreckt, ausgebildet ist, um abgeschnitten zu werden, um eine abgeschrägte distale Oberfläche (347) bereitzustellen, die bündig mit der abgeschrägten Oberfläche (247) der Nadel (204) abschließt.

5. Zugangsbaugruppe nach einem beliebigen Anspruch der Ansprüche 1-4, wobei das flexible Material eine relativ geringe Säulenfestigkeit, eine geringe Scherfestigkeit und eine hohe Druckfestigkeit aufweist; wobei das flexible Material wahlweise eines aus Kunststoff, Polymer, Thermoplast, Polytetrafluorethylen ("PTFE"), Polyethylen ("PE"), Polyurethan ("PET"), Kautschuk, Silikon, Metall, eine Legierung oder Nitinol einschließt.

6. Zugangsbaugruppe (109) nach einem beliebigen Anspruch der Ansprüche 1-6, weiter einschließend einen Lumenräumungsmechanismus, der ausgebildet ist, um den Obturator (104, 304) relativ zum Nadellumen (251) vorzuschieben, um Material aus einem distalen Abschnitt des Nadellumens (251) zu räumen; wobei der Lumenräumungsmechanismus wahlweise automatisch ausgelöst wird, wenn der Obturator (104, 304) aus dem Nadellumen (251) entfernt wird.

7. Verfahren zur Herstellung einer Zugangsbaugruppe, umfassend:
Bereitstellen einer Nadel (204), die von einer Nadelnabe (203) gestützt wird und ein Lumen (251) definiert;
Bereitstellen eines Obturators (104, 304), der von einer Obturatornabe (103) gestützt wird, wobei der Obturator einen radial symmetrischen, länglichen Körper (343) umfasst, der für Einführung in ein Lumen (251) der Nadel ausgelegt ist, **dadurch gekennzeichnet, dass** der längliche Körper (343) verformbar ist, wenn er im Lumen der Nadel (251) angeordnet ist, um sich an die Gestalt eines Innenprofils des distalen Lumens einzupassen; und
das Verfahren weiter Folgendes umfasst:
Vorschieben des Obturators (104, 304) durch das Nadellumen (251), bis sich eine distale Spitze (146) distal von einer distalen Spitze (246) der Nadel erstreckt, wenn die Obturatornabe (103) in die Nadelnabe (203) eingreift; und
Abschneiden eines distalen Abschnitts (342) des Obturators, um eine distale Oberfläche (147) bereitzustellen, die bündig mit einer abgeschrägten distalen Oberfläche (247) der Nadel abschließt.

8. Verfahren nach Anspruch 7, weiter einschließend Befestigen des Obturators (104, 304) an der Obturatornabe (103) vor Vorschieben des Obturators (104, 304) durch das Nadellumen (251).

9. Verfahren nach Anspruch 7 oder 8, wobei der Obturator (104, 304) aus einem flexiblen Material gebildet ist, das eine relativ geringe Säulenfestigkeit, eine geringe Scherfestigkeit und eine hohe Druckfestigkeit aufweist; wobei das flexible Material wahlweise eines aus Kunststoff, Polymer, Thermoplast, Polytetrafluorethylen ("PTFE"), Polyethylen ("PE"), Polyurethan ("PEI"), Kautschuk, Silikon, Metall, eine Legierung oder Nitinol einschließt.

10. Verfahren nach einem beliebigen Anspruch der Ansprüche 7-9, wobei Bereitstellen eines Obturators (104, 304) einschließt, dass der Obturator (104, 304) eine distale Oberfläche (346B) definiert, die sich senkrecht zu einer longitudinalen Achse des Obturators erstreckt.

11. Verfahren nach einem beliebigen Anspruch der Ansprüche 7-10, wobei Bereitstellen eines Obturators (104, 304) einschließt, dass der Obturator (104, 304) eine radial symmetrisch gekrümmte distale Oberfläche (346A) definiert.

12. Verfahren nach einem beliebigen Anspruch der Ansprüche 7-11, weiter einschließend einen Lumenräumungsmechanismus, der ausgebildet ist, um den Obturator (104, 304) relativ zum Nadellumen (251) vorzuschieben, um Material aus einem distalen Abschnitt des Nadellumens (251) zu räumen.

13. Verfahren nach Anspruch 12, wobei der Lumenräumungsmechanismus automatisch ausgelöst wird, wenn die Obturatornabe (103) von der Nadelnabe (203) abgenommen wird.

## Revendications

1. Ensemble d'accès (109) destiné à être utilisé avec un dispositif d'accès intraosseux, comprenant :
une aiguille (204) incluant une région distale arrondie (248) communiquant avec une ouverture distale biseautée (260) ;
un obturateur (104, 304) formé d'un matériau souple, l'obturateur (104, 304) comprenant un corps allongé (343) radialement symétrique conçu pour être inséré dans une lumière (251) de l'aiguille ;
un moyeu d'aiguille (203) configuré pour supporter l'aiguille (204) et définir une lumière de moyeu qui communique avec la lumière d'aiguille (251), et
un moyeu d'obturateur (103) configuré pour supporter l'obturateur (104, 304), **caractérisé en ce qu'**une pointe distale (146) de l'obturateur s'étend à travers la lumière d'aiguille (251) et s'étend distalement au-delà d'une pointe distale (246) de l'aiguille lorsque le moyeu d'obturateur (103) vient en prise avec le moyeu d'aiguille (203) ; et
le corps allongé (343) est déformable lorsqu'il est disposé dans la lumière (251) de l'aiguille pour se conformer afin d'épouser la forme d'un profil interne de la lumière distale.

2. Ensemble d'accès selon la revendication 1, dans lequel l'obturateur (104, 304) comprend une surface distale (346B) s'étendant perpendiculairement à un axe longitudinal.

3. Ensemble d'accès selon l'une quelconque des revendications 1 à 2, dans lequel l'obturateur (104, 304) comprend une surface distale incurvée (346A).

4. Ensemble d'accès selon l'une quelconque des revendications 1 à 3, dans lequel une portion excédentaire de l'obturateur (304) qui s'étend à travers l'ouverture (260), est configurée pour être coupée afin de fournir une surface distale biseautée (347) qui est affleurante à la surface biseautée (247) de l'aiguille (204).

5. Ensemble d'accès selon l'une quelconque des revendications 1 à 4, dans lequel le matériau souple présente une résistance au flambage relativement faible, une faible résistance au cisaillement et une résistance élevée à la compression ; facultativement, dans lequel le matériau souple inclut l'un parmi un plastique, un polymère, un thermoplastique, du polytétrafluoroéthylène (« PTFE »), du polyéthylène (« PE »), du polyuréthane (« PET »), du caoutchouc, du silicone, du métal, un alliage, ou du Nitinol.

6. Ensemble d'accès (109) selon l'une quelconque des revendications 1 à 6, incluant en outre un mécanisme de dégagement de la lumière, configuré pour faire avancer l'obturateur (104, 304) par rapport à la lumière d'aiguille (251) afin de dégager de la matière d'une portion distale de la lumière d'aiguille (251) ; facultativement, dans lequel le mécanisme de dégagement de la lumière est déclenché automatiquement lorsque l'obturateur (104, 304) est retiré de la lumière d'aiguille (251).

7. Procédé de fabrication d'un ensemble d'accès, comprenant les étapes consistant à :
fournir une aiguille (204) supportée par un moyeu d'aiguille (203) et définissant une lumière (251) ;
fournir un obturateur (104, 304) supporté par un moyeu d'obturateur (103), l'obturateur comprenant un corps allongé (343) radialement symétrique conçu pour être inséré dans une lumière (251) de l'aiguille, **caractérisé en ce que** le corps allongé (343) est déformable lorsqu'il est disposé dans la lumière de l'aiguille (251) pour se conformer afin d'épouser la forme d'un profil interne de la lumière distale ; et
le procédé comprend en outre les étapes consistant à :
faire avancer l'obturateur (104, 304) à travers la lumière d'aiguille (251) jusqu'à ce qu'une pointe distale (146) s'étende distalement au-delà d'une pointe distale (246) de l'aiguille, lorsque le moyeu d'obturateur (103) vient en prise avec le moyeu d'aiguille (203) ; et
couper une portion distale (342) de l'obturateur afin de fournir une surface distale (147) qui est au ras de la surface distale biseautée (247) de l'aiguille.

8. Procédé selon la revendication 7, incluant en outre le fait de faire adhérer l'obturateur (104, 304) au moyeu d'obturateur (103) avant de faire avancer l'obturateur (104, 304) à travers la lumière d'aiguille (251).

9. Procédé selon l'une ou l'autre des revendications 7 ou 8, dans lequel l'obturateur (104, 304) est formé d'un matériau souple qui présente une résistance au flambage relativement faible, une faible résistance au cisaillement et une résistance élevée à la compression ; facultativement, dans lequel le matériau souple inclut l'un parmi un plastique, un polymère, un thermoplastique, du polytetrafluoroéthylène (« PTFE »), du polyéthylène (« PE »), du polyuréthane (« PEI »), du caoutchouc, du silicone, du métal, un alliage, ou du Nitinol.

10. Procédé selon l'une quelconque des revendications 7 à 9, dans lequel fournir un obturateur (104, 304) inclut le fait que l'obturateur (104, 304) définisse une surface distale (346B) s'étendant perpendiculairement à un axe longitudinal de l'obturateur.

11. Procédé selon l'une quelconque des revendications 7 à 10, dans lequel fournir un obturateur (104, 304) inclut le fait que l'obturateur (104, 304) définisse une surface distale incurvée (346A) radialement symétrique.

12. Procédé selon l'une quelconque des revendications 7 à 11, incluant en outre un mécanisme de dégagement de la lumière, configuré pour faire avancer l'obturateur (104, 304) par rapport à la lumière d'aiguille (251) afin de dégager le matériau d'une portion distale de la lumière d'aiguille (251).

13. Procédé selon la revendication 12, dans lequel le mécanisme de dégagement de la lumière est déclenché automatiquement lorsque le moyeu d'obturateur (103) est détaché du moyeu d'aiguille (203).
